(19) Europäisches Patentamt
European Patent Office
Office européen des brevets

(11) **EP 2 197 553 B1**

(12) **FASCICULE DE BREVET EUROPEEN**

(45) Date de publication et mention de la délivrance du brevet:
**27.02.2013 Bulletin 2013/09**

(21) Numéro de dépôt: **08867477.5**

(22) Date de dépôt: **17.10.2008**

(51) Int Cl.:
*A61Q 1/14* *(2006.01)*    *A61Q 19/10* *(2006.01)*
*A61K 8/02* *(2006.01)*    *A61K 8/26* *(2006.01)*
*A61K 8/73* *(2006.01)*    *A61K 8/25* *(2006.01)*

(86) Numéro de dépôt international:
**PCT/FR2008/001465**

(87) Numéro de publication internationale:
**WO 2009/083671 (09.07.2009 Gazette 2009/28)**

(54) **PROCEDE DE FABRICATION D'UN ARTICLE DE NETTOYAGE ET/OU DE SOIN**

VERFAHREN ZUR HERSTELLUNG EINES REINIGUNGS- UND/ODER PFLEGEARTIKELS

METHOD OF MANUFACTURING A CLEANING AND/OR CARE ARTICLE

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **17.10.2007 FR 0707254**

(43) Date de publication de la demande:
**23.06.2010 Bulletin 2010/25**

(73) Titulaire: **Georgia-Pacific France**
**92270 Bois-Colombes (FR)**

(72) Inventeurs:
• **CLERMONT, Anne-Gaëlle**
  **F-68000 Colmar (FR)**
• **FLORENCE, Jocelyne**
  **F-68650 Lapoutroie (FR)**
• **BRET, Bruno**
  **F-68920 Wintzenheim (FR)**

(74) Mandataire: **Noel, Chantal Odile et al**
**Cabinet Orès**
**36, rue de St Pétersbourg**
**75008 Paris (FR)**

(56) Documents cités:
| | |
|---|---|
| EP-A1- 1 043 019 | EP-A1- 1 374 834 |
| EP-A2- 0 102 200 | WO-A1-02/24849 |
| WO-A1-03/105804 | WO-A2-96/36758 |
| US-A- 3 623 990 | US-A- 3 636 200 |
| US-A- 4 482 538 | US-A- 4 833 003 |
| US-A1- 2004 228 811 | US-A1- 2005 175 642 |

**Description**

L'invention concerne l'utilisation d'une composition de nettoyage et/ou de soin, comprenant au moins un composé de nettoyage et/ou de soin, visqueuse, rhéofluidifiante et thixotrope pour l'imprégnation d'une pile de supports absorbants, ainsi qu'un procédé de fabrication d'articles de nettoyage et/ou de soin.

Elle décrit également une composition de nettoyage et/ou de soin ainsi qu'un article de nettoyage et/ou de soin.

Il existe à l'heure actuelle de nombreux supports préimprégnés, du type lingettes humides, sur lesquels des produits de nettoyage et/ou de soin ont été préalablement appliqués, qui sont utilisés en lieu et place des traditionnels tampons secs, généralement en coton, que l'utilisateur imprègne au moment de leur utilisation.

Ainsi, le produit de nettoyage et/ou de soin peut être un produit de nettoyage de lunettes, de vitres, de sols carrelés ou en parquet, un produit lustrant pour les meubles, un produit à base de cire pour l'entretien et le nettoyage du bois, un produit de nettoyage des plans de travail dans une cuisine, ou encore un produit du type cirage pour le nettoyage et le soin des cuirs.

Un domaine particulièrement concerné par l'utilisation de tels supports imprégnés est le domaine des produits d'hygiène corporelle comprenant un produit de nettoyage et/ou de soin de la peau, en particulier du type démaquillant, ou de nettoyage et/ou de soin de la peau des bébés.

Dans tous ces domaines d'utilisation, ces supports préimprégnés évitent, d'une part, de transporter et de manipuler des récipients additionnels contenant les produits à imprégner et permettent, d'autre part, de délivrer uniquement la quantité nécessaire à l'utilisation envisagée.

Les procédés d'obtention de ces supports préimprégnés se démarquent essentiellement des procédés traditionnels utilisés jusqu'à présent pour fabriquer le support de base en ce qu'ils prévoient une étape d'imprégnation des supports avec une composition adaptée à l'utilisation finale desdits supports.

Dans le cas d'une utilisation cosmétique, notamment le démaquillage du visage, les produits d'imprégnation sont généralement des lotions aqueuses ou hydroalcooliques, ou des émulsions fluides huile-dans-eau.

Mais les technologies actuelles d'imprégnation, telles que la pulvérisation ou le trempage, ne permettent pas, de mouiller de manière suffisamment homogène et fiable des supports lorsque les produits sont trop visqueux.

Cet inconvénient est particulièrement prononcé lorsque l'on imprègne une pile de supports au moyen d'une seule injection de liquide au sommet de la pile.

On constate notamment que le liquide visqueux ne diffuse pratiquement pas dans les supports disposés dans la partie basse de la pile.

En outre, pour les supports disposés dans la partie haute, l'imprégnation ne se fait pas de manière homogène, le liquide se répartissant uniquement sur certaines zones des supports, les autres zones en étant totalement dépourvues.

Pour résoudre le problème évoqué ci-dessus, les solutions envisagées jusqu'à présent ont consisté soit à se limiter à l'imprégnation de liquides peu visqueux, soit à imprégner les supports individuellement et non plus en pile.

Ces solutions s'avèrent toutefois peu satisfaisantes.

Dans le premier cas, on limite de manière exagérée la gamme des produits potentiellement utilisables à ce niveau.

Ainsi, dans certaines applications cosmétiques, où le caractère crémeux et onctueux de la composition cosmétique est très important aux yeux des utilisateurs, la limitation à l'imprégnation de liquide peu visqueux ne permet pas de proposer de tels produits en lingette pré-imprégnée.

En outre, l'imprégnation de compositions fluides dans une pile de supports absorbants, comme des tampons en coton, engendre généralement une diffusion progressive dans le temps et du haut vers le bas de la composition dans la pile : la composition n'est donc plus répartie de manière homogène dans la pile lors d'un stockage relativement long.

Dans le second cas, on est obligé d'utiliser une technologie particulièrement complexe et coûteuse pour assurer une bonne imprégnation de chacun des supports, tout en diminuant de manière sensible les rendements de production par comparaison avec ceux obtenus lors d'une imprégnation en pile.

L'invention vise donc à résoudre les problèmes soulevés par cet art antérieur.

A cet effet, l'objet de l'invention est l'utilisation d'une composition de nettoyage et/ou de soin, comprenant au moins un composé de nettoyage et/ou de soin, ladite composition ayant une viscosité apparente mesurée à 20°C selon la norme ASTM D 2983 supérieure à 80 centipoises, de préférence supérieure à 200 centipoises plus préférablement supérieure à 500 centipoises et étant rhéofluidifiante et thixotrope, pour l'imprégnation, sous contrainte, d'une pile de supports absorbants.

Plus préférablement, la composition de nettoyage et/ou de soin utilisée a une viscosité apparente mesurée à 20°C selon la norme ASTM D 2983 supérieure à 2 500 centipoises, de préférence supérieure à 12 500 centipoises.

De préférence, la composition de nettoyage et/ou de soin utilisée est assimilable à un corps plastique de Bingham jusqu'à ce que sa valeur atteigne une valeur seuil, lorsqu'elle est soumise à un taux de cisaillement donné.

Plus préférablement, la composition de nettoyage et/ou de soin utilisée a une viscosité $\eta$ décroissant exponentiellement selon l'expression :

$$B' = - (d \eta / d t).t,$$

le coefficient B étant supérieur ou égal à 0,4, de préférence supérieur ou égal à 4,
lorsqu'elle est soumise à un taux de cisaillement de 20 tours/min au moyen d'un appareil Brookfield muni d'un mobile référence SC4-31 et jusqu'à l'obtention d'une valeur seuil de viscosité.

Encore plus préférablement, la composition de nettoyage et/ou de soin utilisée, lorsqu'elle est soumise à un taux de cisaillement de 20 tours/min au moyen d'un appareil Brookfield muni d'un mobile référence SC4-31 et jusqu'à l'obtention d'une valeur seuil de viscosité, a une viscosité η décroissant exponentiellement selon l'expression :

$$B = - (d \eta / d t).t,$$

le coefficient B étant compris entre 15 et 300, de préférence compris entre 200 et 300.

Toujours de préférence, la composition de nettoyage et/ou de soin utilisée a un taux de reprise TR inférieur ou égal à 50 %, de préférence compris entre 20 % et 30%.

Dans un mode de mise en oeuvre préféré de l'utilisation de l'invention, la composition de nettoyage et/ou de soin utilisée comprend du magnésium aluminium silicate et du sodium carboxyméthylcellulose.

Dans ce cas, de préférence, la composition de nettoyage et/ou de soin comprend au moins 0,4 %, de préférence au moins 0,7 %, plus préférablement au moins 0,8% en poids, par rapport au poids total de la composition, de magnésium aluminium silicate et au moins 0,12 %, de préférence au moins 0,21 %, plus préférablement au moins 0,24 %, en poids, par rapport au poids total de ladite composition, de sodium carboxyméthylcellulose.

Dans un mode de mise en oeuvre tout particulièrement préféré de l'utilisation de l'invention, le au moins un composé de nettoyage et/ou de soin est au moins un composé de nettoyage et/ou de soin de la peau.

Est également décrit un procédé de fabrication d'articles de nettoyage et/ou de soin comprenant les étapes suivantes :

a) empilement l'un sur l'autre de plusieurs supports absorbants, et
b) application sous contrainte d'une composition de nettoyage et/ou de soin, comprenant au moins un composé de nettoyage et/ou de soin, ladite composition ayant une viscosité apparente mesurée à 20°C selon la norme ASTM D 2983 supérieure à 80 centipoises, et de préférence supérieure à 200 centipoises et étant rhéofluidifiante et thixotrope, sur le support absorbant disposé sur le haut de l'empilement.

Plus préférablement, dans ce procédé à l'étape b), la composition de soin et/ou de nettoyage a une viscosité apparente mesurée à 20°C selon la norme ASTM D 2983 supérieure à 500 centipoises, de préférence supérieure à 2 500 centipoises, le plus préférablement supérieure à 12 500 centipoises.

De préférence, dans ce procédé, à l'étape b), la composition de nettoyage et/ou de soin est assimilable à un corps plastique de Bingham jusqu'à ce que sa viscosité atteigne une valeur seuil.

Plus préférablement, dans ce procédé, à l'étape b), 1a composition de nettoyage et/ou de soin, lorsqu'elle est soumise à un taux de cisaillement de 20 tours/min au moyen d'un appareil Brookfield muni d'un mobile référence SC4-31 et jusqu'à l'obtention d'une valeur seuil de viscosité, a une viscosité η décroissant exponentlellement selon l'expression :

$$B = - (d \eta / d t).t,$$

le coefficient B étant supérieur ou égal à 0,4, de préférence supérieur ou égal à 4.

Encore plus préférablement, dans ce procédé, à l'étape b), la composition de nettoyage et/ou de soin, lorsqu'elle est soumise à un taux de cisaillement de 20 tours/min au moyen d'un appareil Brookfield muni d'un mobile référence SC4-31 et jusqu'à l'obtention d'une valeur seuil de viscosité, a une viscosité η décroissant exponentiellement selon l'expression :

$$B = - (d \eta / d t).t,$$

le coefficient B étant compris entre 15 et 300, de préférence, entre 200 et 300.

De préférence, dans ce procédé, à l'étape b), la composition de soin et/ou de nettoyage appliquée possède un taux de reprise TR inférieur ou égal à 50 %, de préférence compris entre 20 et 30 %.

Dans un mode de mise en oeuvre préféré de ce procédé, à l'étape b), la composition de nettoyage et/ou de soin comprend du magnésium aluminium silicate et du sodium carboxyméthylcellulose.

Dans ce cas, de préférence, à l'étape b), la composition de soin et/ou de nettoyage comprend au moins 0,4 % en poids, par rapport au poids total de la composition, de magnésium aluminium silicate et au moins 0,12 % en poids, par rapport au poids total de la composition, de sodium carboxyméthylcellulose.

Plus préférablement, à l'étape b), la composition de nettoyage et/ou de soin comprend au moins 0,7 % en poids par rapport au poids total de la composition, de magnésium aluminium silicate et au moins 0,21 % en poids, par rapport au poids total de la composition, de sodium carboxyméthylcellulose.

Encore plus, à l'étape b), la composition de nettoyage et/ou de soin comprend au moins 0,8 % en poids, par rapport au poids total de la composition, de magnésium aluminium silicate et au moins 0,24 % en poids, par rapport au poids total de la composition, de sodium carboxyméthylcellulose.

Une composition de nettoyage et/ou de soin particulièrement adaptée pour l'utilisation selon l'invention et pour la mise en oeuvre du procédé de fabrication décrit ici est une composition comprenant au moins un composé de nettoyage et/ou de soin ayant un comportement assimilable à celui d'un corps plastique de Bingham jusqu'à ce que sa viscosité atteigne une valeur seuil, et une viscosité apparente mesurée à 20°C selon la norme ASTM D 2983 supérieure à 80 centipoises, plus préférablement supérieure à 200 centipoises, encore plus préférablement supérieure à 500 centipoises.

De préférence, cette composition de nettoyage et/ou de soin a une viscosité apparente mesurée à 20°C selon la norme ASTM D 2983 supérieure à 2 500 centipoises, plus préférablement supérieure à 12 500 centipoises.

De préférence, cette composition de nettoyage et/ou de soin, lorsqu'elle est soumise à un taux de cisaillement de 20 tours/min au moyen d'un appareil Brookfield muni d'un mobile référence SC4-31 et jusqu'à l'obtention d'une valeur seuil de viscosité, a une viscosité $\eta$ décroissant exponentiellement selon l'expression :

$$B = - (d\,\eta\,/\,d\,t).t,$$

le coefficient B étant supérieur ou égal à 0,4, de préférence supérieur ou égal à 4.

Plus préférablement, cette composition de nettoyage et/ou de soin lorsqu'elle est soumise à un taux de cisaillement de 20 tours/min au moyen d'un appareil Brookfield muni d'un mobile référence SC4-31 et jusqu'à l'obtention d'une valeur seuil de viscosité, a une viscosité $\eta$ décroissant exponentiellement selon l'expression :

$$B = - (d\,\eta\,/\,d\,t).t$$

le coefficient B étant compris entre 15 et 300, de préférence, entre 200 et 300.

De préférence, cette composition de nettoyage et/ou de soin a un taux de reprise TR inférieur ou égal à 50 %, de préférence, compris entre 20 et 30 %.

Dans un mode de réalisation préféré, cette composition de nettoyage et/ou de soin comprend du magnésium aluminium silicate et du sodium carboxyméthylcellulose.

Dans ce cas, elle comprend au moins 0,4 %, de préférence au moins 0,7 %, plus préférablement au moins 0,8 %, en poids par rapport au poids total de ladite composition, de magnésium aluminium silicate et au moins 0,12 %, plus préférablement au moins 0,21 %, encore plus préférablement au moins 0,24 % en poids, par rapport au poids total de ladite composition, de sodium carboxyméthylcellulose.

Dans un mode de réalisation particulièrement préféré, cette composition de nettoyage et/ou de soin comprend le au moins un composé de nettoyage et/ou de soin de la peau.

Un article de nettoyage et/ou de soin est également décrit. Il est constitué d'un support absorbant imprégné d'une composition de nettoyage et/ou de soin selon l'invention et qui libère un taux de composition de nettoyage et/ou de soin selon l'invention supérieur à 20 % pour un temps de pression de 120 secondes.

De préférence, dans cet article, le support absorbant est un support en coton.

L'invention repose sur l'utilisation d'une composition de nettoyage et/ou de soin, comprenant au moins un composé de nettoyage et/ou de soin, visqueuse tout en ayant un comportement rhéologique de type rhéofluidifiant et thixotrope.

Dans l'invention, par composition visqueuse, on entend une composition dont la viscosité apparente déterminée au moyen du viscosimètre Brookfield, qui mesure le couple nécessaire pour faire tourner un mobile Brookfield n°2 à une vitesse constante de 12 tours/min dans un bain de ladite composition à une température donnée de 20°C et conformément à la norme ASTM D 2983, comprise entre 80 et 40 000 centipoises.

Le composé de nettoyage et/ou de soin peut être une cire pour le nettoyage et le soin des bois, un produit de nettoyage des vitres, des lunettes, des sols carrelés ou en parquet. De préférence, le au moins un composé de nettoyage et/ou de soin est un composé de nettoyage et/ou de soin de la peau, plus particulièrement de la peau des bébés.

En utilisant une telle composition de nettoyage et/ou de soin, lors d'une imprégnation avec contrainte, notamment sous pression et avec agitation préalable, la composition se fluidifie suffisamment pour permettre sa diffusion rapide et homogène dans des supports disposés en pile.

Par ailleurs, du fait de sa thixotropie, la composition ne reprend pas instantanément sa viscosité initiale lorsqu'elle est à nouveau au repos.

Ce temps plus ou moins long permet d'améliorer encore la diffusion de la composition à travers les supports et au sein de chacun des supports.

En outre, lorsque la composition a repris sa viscosité initiale, elle ne migre plus à travers les supports comme le ferait une composition fluide : le phénomène de diffusion dans le temps évoqué précédemment pour les compositions fluides ne se produit donc plus.

Dans l'invention, par composition visqueuse, on entend une composition dont la viscosité apparente est comprise entre 80 et 40 000 centipoises, déterminée au moyen du viscosimètre Brookfield, qui mesure le couple nécessaire pour faire tourner un mobile Brookfield n°2 à une vitesse constante de 12 tours/min dans un bain de ladite composition à une température donnée de 20°C et conformément à la norme ASTM D 2983.

D'autres avantages et caractéristiques ressortiront mieux de la description qui va suivre de quelques exemples de réalisation selon l'invention.

EXEMPLE 1 :

On prépare une composition cosmétique contenant les ingrédients suivants :

- 0,3 % de magnésium aluminium silicate
- 0,09 % de carboxyméthylcellulose
- 0,1% de tétrasodium EDTA
- 0,25 % de chlorphénésine
- 3,0 % de glycérine
- 0,9 % d'un mélange de butylparabène, de propylparabène, d'isobutylparabène, de phénoxyéthanol, de méthylparabène et d'éthylparabène
- 3,0 % d'un mélange de cetéareth-20, de cétéareth-12, de alcool cétéarylique, de palmitate de cétyle et de stearate de glycéryle
- 1,0 % de cétéareth-20
- 6,0 % d'une huile minérale
- 2,0 % d'isohexadécane
- 1,0 % de caprylic/capric triglycéride
- 1,0 % de polydiméthylsiloxane
- 0,15 % de parfum
- 1,0 % d'un mélange de glycérine, de butylène glycol, d'eau et d'extraits végétaux
- 80,2 % d'eau

On a déterminé les caractéristiques rhéologiques de cette composition en mesurant d'abord sa viscosité apparente en centipoises, déterminée au moyen du viscosimètre Brookfield, qui mesure le couple nécessaire pour faire tourner un mobile Brookfield n°2 à une vitesse constante de 12 tours/min dans un bain de ladite composition à une température donnée de 20°C et conformément à la norme ASTM D 2983.

La viscosité mesurée est de 85 cP.

On a ensuite caractérisé le comportement rhéologique de cette composition, notamment ses variations de viscosité en fonction du temps, lorsqu'elle est soumise à une contrainte de cisaillement constante.

On a pour cela utilisé un appareil Brookfield, commercialisé sous la référence Rheocalc data1 LV, muni d'un mobile référence SC4-31 tournant à 20 tours/min.

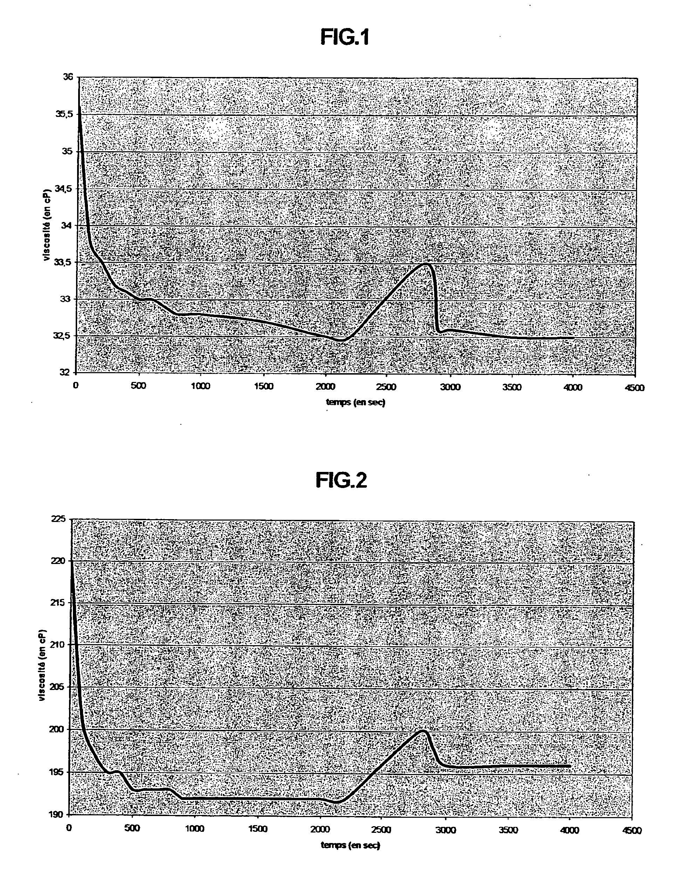

Les valeurs de viscosité relevées à ce niveau ont été regroupées dans le tableau 1 et le graphique correspondant a été représenté sur la figure 1.

On constate tout d'abord que la viscosité diminue en fonction du temps selon une loi sensiblement exponentielle avant d'atteindre une valeur seuil.

La composition visqueuse a donc un comportement rhéofluidifiant.

A ce titre, jusqu'à ce que sa viscosité atteigne la valeur seuil, elle est assimilable, à un corps plastique de Bingham pour lequel la viscosité $\eta$ de la composition décroît exponentiellement selon l'expression :

$$B = - (d \, \eta \, / \, d \, t) \, . \, t,$$

B étant le coefficient temporel de destruction thixotropique.

On détermine par le calcul et par extrapolation que B est égal, dans ce cas, à 0,40.

Pour caractériser la thixotropie de cette composition, on a par ailleurs stoppé la contrainte de cisaillement, de préférence, lorsque la viscosité de la composition a déjà atteint sa valeur seuil $\eta 1$, avant de reprendre cette même contrainte après un temps d'attente de 10 minutes.

On remarque dans le tableau 1 et sur la figure 1 que la viscosité $\eta 2$ de la composition mesurée juste avant de reprendre la contrainte de cisaillement est sensiblement inférieure à la viscosité $\eta 0$ de la composition au départ.

En effet, la reprise de viscosité d'une composition thixotrope n'est pas immédiate après arrêt de la contrainte de cisaillement.

Pour caractériser ce phénomène, on a évalué le taux de reprise TR de cette composition après un temps de repos de 10 minutes, lequel correspond à :

$$TR = (\eta 2 - \eta 1)/(\eta 0 - \eta 1) X 100,$$

Ce qui donne, dans ce cas, un taux de reprise TR de 30 % environ.

Plus le taux de reprise TR est faible, plus la composition est thixotrope.

On a ensuite évalué le pouvoir d'imprégnation amélioré de cette composition.

On a pour cela imprégné un empilement de cinq tampons à démaquiller de 120 g/m$^2$ constitués de cent pour cent de fibres de coton, fabriqués par la demanderesse à partir de nappes décrites dans le brevet européen n°0 681 621, en déposant une quantité de ladite composition sur le tampon disposé sur le dessus de la pile. La quantité de composition déposée a été calculée pour correspondre à une imprégnation finale de 1 g par gramme de coton.

Deux séries de mesures ont été effectuées.

Dans la première série, on a laissé diffuser la composition à travers la pile de tampons de coton sans appliquer de contrainte.

Dans la deuxième série, on applique une contrainte continue sur la pile juste après le dépôt de la composition au moyen d'un poids de 5,2 kg.

Dans chacune des séries de mesures, on attend 5 minutes après la dépose et on pèse chacun des tampons en coton.

Connaissant le poids du tampon au départ, on peut calculer le poids de composition absorbée à l'intérieur du coton, puis le pourcentage de la composition totale que cela représente.

Les résultats ont été regroupés dans le tableau 2.

On constate que la composition diffuse mieux à l'intérieur de la pile lorsqu'elle est soumise à une contrainte.

Ceci résulte évidemment du comportement rhéofluidifiant de la composition, lequel induit une baisse de viscosité en cas de contrainte et, de ce fait, améliore la diffusion dans la pile.

On a également évalué la capacité pour un tampon en coton imprégné de ladite composition à relarguer sous contrainte cette composition sur une surface de transfert en contact avec ledit tampon en coton.

Pour cela, on a imprégné des tampons démaquiller de 120 g/m$^2$ constitués de cent pour cent de fibres de coton, fabriqués par la demanderesse à partir de nappes décrites dans le brevet européen n°0 681 621, avec la composition précédente et selon un taux d'environ 4 grammes de composition par gramme de coton. Puis, on a mesuré la quantité de composition restituée par application d'une charge sur le tampon.

Le mode opératoire est le suivant :

1) on pèse le tampon imprégné de la composition à l'aide d'une balance à 0,01 g près : on détermine ainsi le poids M1 ;

2) on prépare du papier buvard (Whatman 201 (réf 5201-930)) découpé (diamètre 112 mm ou carré de 145 mm de côté) avec un emporte-pièce ou au ciseau;

3) on pose le tampon imprégné sur les 10 couches de buvard et on le recouvre de 10 autres couches de buvard ;

4) on pose une charge de 3,5 kg sur le sandwich de matière obtenu pendant un temps déterminé t. Le tampon possédant une surface d'environ 25,5 cm2 (disque de diamètre 57 mm), la pression moyenne appliquée sur celui-ci par la charge vaut environ 138 g/cm2. Cette valeur correspond à une pression relativement faible, normalement inférieure à la pression généralement exercée par les doigts sur le visage lors de l'opération sur le visage.

5) On retire la charge et on pèse le tampon : la différence entre le poids M1 du tampon avant compression et le poids M2 du tampon après compression détermine la quantité de lotion extraite.

6) On rince le tampon à l'eau chaude pour éliminer le reste de lotion, puis on le laisse sécher à l'étuve pendant 2 heures à 100°C.

7) On mesure le poids M3 du tampon à sec

8) On détermine ainsi le taux d'imprégnation du tampon au départ du test :

$$TI = (M1-M3)/M3,$$

ainsi que le taux de composition libérée par le format ( taux de relarguage) :

$$TL = (M1-M2)/(M1-M3)$$

Les résultats ont été regroupés dans le tableau 3.

Les mesures ont été faites pour des temps de compression respectifs de 10, 30, 60 et 120 secondes.

On constate que, même pour un temps de compression de 10 secondes, le taux de relarguage est supérieure à 20 %, ce qui correspond au taux de relarguage moyen mesuré sur des tampons en coton gratté du commerce de grammage égal à 100 g/m$^2$ et soumis à une pression de 300 g/cm$^2$ pendant 60 secondes.

En outre, ce taux de lotion libérée par le tampon augmente rapidement avec le temps de compression du fait du comportement rhéofluidifiant de la composition.

En particulier, on constate que le taux de relarguage pour un temps de compression de 120 secondes est de 40,6 %.

Ceci confère au final au tampon en coton imprégné, dans les conditions normales de son utilisation, de très bonnes caractéristiques de libération du produit cosmétique.

Par ailleurs, ces caractéristiques sont obtenues sans que l'utilisateur ait besoin d'exercer une pression forte sur son visage, ce qui constitue un avantage supplémentaire de l'invention.

EXEMPLE 2 :

On prépare une composition cosmétique contenant les ingrédients suivants :

- 0,4 % de magnésium aluminium silicate
- 0,12 % de carboxyméthylcellulose
- 0,1 % de tétrasodium EDTA
- 0,2 % de chlorphénésine
- 3,0 % de glycérine
- 0,8 % d'un mélange de butylparabène, de propylparabène, d'isobutylparabène, de phénoxyéthanol, de méthylparabène et d'éthylparabène
- 3,0 % d'un mélange de cétéareth-20, de cétéareth-12, de alcool cétéarylique, de palmitate de cétyle et de stéarate de glycéryle
- 1,0 % de cétéareth-20
- 6,0 % d'une huile minérale
- 2,0 % d'isohexadécane
- 1,0 % de caprylic/capric triglycéride
- 1,0 % de polydiméthylsiloxane
- 0,15 % de parfum
- 1,0 % d'un mélange de glycérine, de butylène glycol, d'eau et d'extraits végétaux
- 80,23 % d'eau

On a ensuite soumis cette composition aux mêmes mesures que l'exemple 1 précédemment décrit.

On détermine ainsi une viscosité apparente pour la composition de 525cP.

En mesurant les variations de viscosité en fonction du temps, lorsque la composition est soumise à une contrainte de cisaillement constante, valeurs regroupées dans le tableau 1 et représentées sur la figure 2, on constate que la composition a une comportement rhéofluidifiant et est assimilable à un corps plastique de Bingham, jusqu'à ce que sa viscosité atteigne une valeur seuil.

On détermine dans ce cas, par le calcul, que la composition possède un coefficient temporel de destruction thixotropique B égal à 4,34 et un taux de reprise TR égal à 28 %.

On a ensuite évalué le pouvoir d'imprégnation amélioré de cette composition.

Les résultats ont été regroupés dans le tableau 2.

On constate, comme dans l'exemple 1, que la composition diffuse mieux à l'intérieur de la pile lorsqu'elle est soumise à une contrainte.

On a également évalué la capacité pour un tampon en coton imprégné de ladite composition à relarguer sous contrainte cette composition sur une surface de transfert en contact avec ledit tampon en coton.

Les résultats ont été regroupés dans le tableau 3.

On constate que, même si le taux de relarguage pour un temps de compression de 10 secondes est inférieure à 20 %, ce qui correspond au taux de relarguage moyen mesuré sur des tampons en coton gratté du commerce de grammage égal à 100 g/m$^2$ et soumis à une pression de 300 g/cm$^2$ pendant 60 secondes, le taux de lotion libérée par le tampon augmente rapidement avec le temps de compression du fait du comportement rhéofluidifiant de la composition.

En particulier, on constate que le taux de relarguage pour un temps de compression de 120 secondes est d'au moins 30 %.

EXEMPLE 3:

On prépare une composition cosmétique contenant les ingrédients suivants :

- 0,7 % de magnésium aluminium silicate
- 0,21 % de carboxyméthylcellulose
- 0,1 % de tétrasodium EDTA
- 0,2 % de chlorphénésine
- 3,0 % de glycérine
- 0,8 % d'un mélange de butylparabène, de propylparabène, d'isobutylparabène, de phénoxyéthanol, de méthylparabène et d'éthylparabène
- 3,0 % d'un mélange de cétéareth-20, de cétéareth-12, de alcool cétéarylique, de palmitate de cétyle et de stéarate de glycéryle
- 1,0 % de cétéareth-20
- 6,0 % d'une huile minérale
- 2,0 % d'isohexadécane
- 1,0 % de caprylic/capric triglycéride
- 1,0 % de polydiméthylsiloxane
- 0,15 % de parfum
- 1,0 % d'un mélange de glycérine, de butylène glycol, d'eau et d'extraits végétaux
- 79,84 % d'eau

On a ensuite soumis cette composition aux mêmes mesures que l'exemple 1 précédemment décrit.

On détermine ainsi une viscosité apparente pour la composition de 2645 cP.

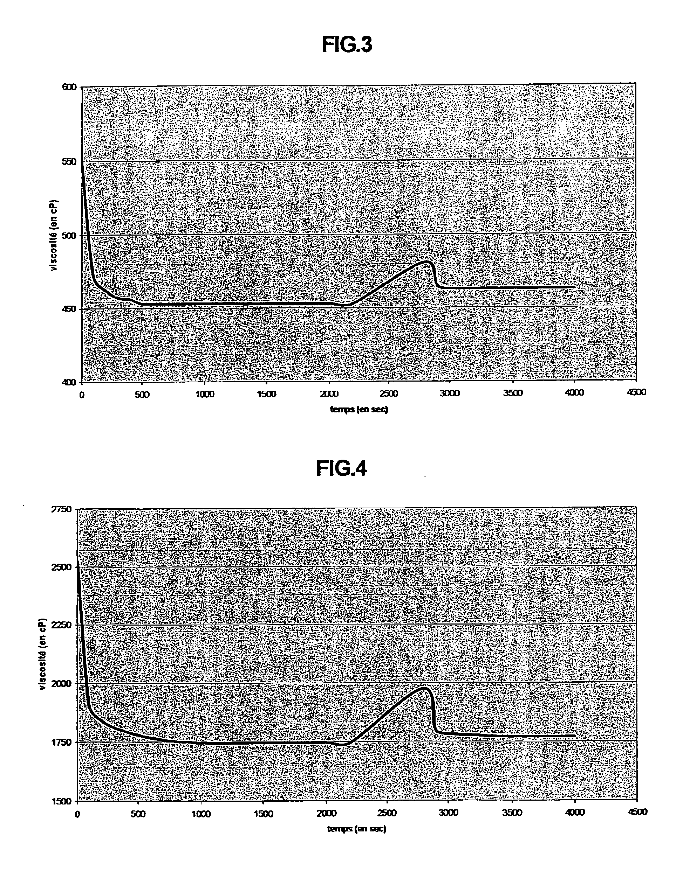

En mesurant les variations de viscosité en fonction du temps, lorsque la composition est soumise à une contrainte de cisaillement constante, valeurs regroupées dans le tableau 1 et représentées sur la figure 3, on constate que la composition a un comportement rhéofluidifiant et est assimilable à un corps plastique de Bingham, jusqu'à ce que sa viscosité atteigne une valeur seuil.

On détermine dans ce cas, par le calcul, que la composition possède un coefficient temporel de destruction thixotropique B égal à 14,96 et un taux de reprise TR égal à 29%.

On a ensuite évalué le pouvoir d'imprégnation amélioré de cette composition.

Les résultats ont été regroupés dans le tableau 2.

On constate, comme dans l'exemple 1, que la composition diffuse mieux à l'intérieur de la pile lorsqu'elle est soumise à une contrainte.

On a également évalué la capacité pour un tampon en coton imprégné de ladite composition à relarguer sous contrainte cette composition sur une surface de transfert en contact avec ledit tampon en coton.

Les résultats ont été regroupés dans le tableau 3.

On constate que, même si le taux de relarguage pour un temps de compression de 10 secondes est inférieure à 20 %, ce qui correspond au taux de relarguage moyen mesuré sur des tampons en coton gratté du commerce de grammage égal à 100 g/m$^2$ et soumis à une pression de 300 g/cm$^2$ pendant 60 secondes, le taux de lotion libérée par le tampon augmente rapidement avec le temps de compression du fait du comportement rhéofluidifiant de la composition.

En particulier, on constate que le taux de relarguage pour un temps de compression de 120 secondes est d'au moins 30 %.

EXEMPLE 4 :

On prépare une composition cosmétique contenant les ingrédients suivants :

- 0,8 % de magnésium aluminium silicate
- 0,24 % de carboxyméthylcellulose
- 0,1 % de tétrasodium EDTA
- 0,2 % de chlorphénésine
- 3,0 % de glycérine
- 0,8 % d'un mélange de butylparabène, de propylparabène, d'isobutylparabène, de phénoxyéthanol, de méthylparabène et d'éthylparabène
- 3,0 % d'un mélange de cétéareth-20, de cétéareth-12, de alcool cétéarylique, de palmitate de cétyle et de stéarate de glycéryle
- 1,0 % de cétéareth-20
- 6,0 % d'une huile minérale
- 2,0 % d'isohexadécane
- 1,0 % de caprylic/capric triglycéride
- 1,0 % de polydiméthylsiloxane
- 0,15 % de parfum
- 1,0 % d'un mélange de glycérine, de butylène glycol, d'eau et d'extraits végétaux
- 79,71 % d'eau

On a ensuite soumis cette composition aux mêmes mesures que l'exemple 1 précédemment décrit.

On détermine ainsi une viscosité apparente pour la composition de 12500 cP.

En mesurant les variations de viscosité en fonction du temps, lorsque la composition est soumise à une contrainte de cisaillement constante, valeurs regroupées dans le tableau 1 et représentées sur la figure 4, on constate que la composition a une comportement rhéofluidifiant et est assimilable à un corps plastique de Bingham, jusqu'à ce que sa viscosité atteigne une valeur seuil.

On détermine dans ce cas, par le calcul, que la composition possède un coefficient temporel de destruction thixotropique B égal à 108,57 et un taux de reprise TR égal à 29 %.

On a ensuite évalué le pouvoir d'imprégnation amélioré de cette composition.

Les résultats ont été regroupés dans le tableau 2.

On constate, comme dans l'exemple 1, que la composition diffuse mieux à l'intérieur de la pile lorsqu'elle est soumise à une contrainte.

On a également évalué la capacité pour un tampon en coton imprégné de ladite composition à relarguer sous contrainte cette composition sur une surface de transfert en contact avec ledit tampon en coton.

Les résultats ont été regroupés dans le tableau 3.

On constate que, même si le taux de relarguage pour un temps de compression de 10 secondes est inférieure à 20 %, ce qui correspond au taux de relarguage moyen mesuré sur des tampons en coton gratté du commerce de grammage égal à 100 g/m$^2$ et soumis à une pression de 300 g/cm$^2$ pendant 60 secondes, le taux de lotion libérée par le tampon augmente rapidement avec le temps de compression du fait du comportement rhéofluidifiant de la composition.

En particulier, on constate que le taux de relarguage pour un temps de compression de 120 secondes est d'au moins 25 %.

EXEMPLE 5 :

On prépare une composition cosmétique contenant les ingrédients suivants :

- 1,6 % de magnésium aluminium silicate
- 0,48 % de carboxyméthylcellulose
- 0,1 % de tétrasodium EDTA
- 0,2 % de chlorphénésine
- 3,0 % de glycérine
- 0,8 % d'un mélange de butylparabène, de propylparabène, d'isobutylparabène, de phénoxyéthanol, de méthylparabène et d'éthylparabène
- 3,0 % d'un mélange de cétéareth-20, de cétéareth-12, de alcool cétéarylique, de palmitate de cétyle et de stéarate de glycéryle
- 1,0 % de cétéareth-20

- 6,0 % d'une huile minérale
- 2,0 % d'isohexadécane
- 1,0 % de caprylic/capric triglycéride
- 1,0 % de polydiméthylsiloxane
- 0,15 % de parfum
- 1,0 % d'un mélange de glycérine, de butylène glycol, d'eau et d'extraits végétaux
- 78,67 % d'eau

On a ensuite soumis cette composition aux mêmes mesures que l'exemple 1 précédemment décrit.

On détermine ainsi une viscosité apparente pour la composition de 24000 cP.

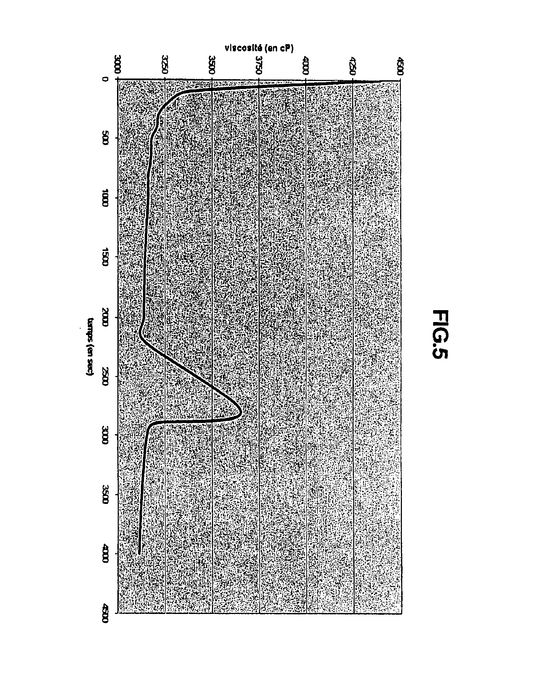

En mesurant les variations de viscosité en fonction du temps, lorsque la composition est soumise à une contrainte de cisaillement constante, valeurs regroupées dans le tableau 1 et représentées sur la figure 5, on constate que la composition a une comportement rhéofluidifiant et est assimilable à un corps plastique de Bingham au moins durant les premières secondes de mesure.

On détermine ainsi, par le calcul, que la composition possède un coefficient temporel de destruction thixotropique B égal à 209,18 et un taux de reprise TR égal à 41 %.

On a ensuite évalué le pouvoir d'imprégnation amélioré de cette composition.

Les résultats ont été regroupés dans le tableau 2.

On constate, comme dans l'exemple 1, que la composition diffuse mieux à l'intérieur de la pile lorsqu'elle est soumise à une contrainte.

On a également évalué la capacité pour un tampon en coton imprégné de ladite composition à relarguer sous contrainte cette composition sur une surface de transfert en contact avec ledit tampon en coton.

Les résultats ont été regroupés dans le tableau 3.

En particulier, on constate que le taux de relarguage pour un temps de compression de 120 secondes est d'au moins 21 %.

TABLEAU 1 :

| | Exemple 1 | Exemple 2 | Exemple 3 | Exemple 4 | Exemple 5 |
|---|---|---|---|---|---|
| Temps (en sec) | Viscosité (en cP) | Viscosité (en cP) | Viscosité (en cP) | Viscosité (en cP) | Viscosité (en cP) |
| 0 | 35,6 | 220 | 549 | 2525 | 4397 |
| 100 | 33,8 | 201 | 472 | 1908 | 3371 |
| 200 | 33,5 | 197 | 462 | 1842 | 3263 |
| 300 | 33,2 | 195 | 457 | 1812 | 3215 |
| 400 | 33,1 | 195 | 456 | 1794 | 3208 |
| 500 | 33,0 | 193 | 453 | 1780 | 3179 |
| 600 | 33,0 | 193 | 453 | 1768 | 3178 |
| 700 | 32,9 | 193 | 453 | 1758 | 3173 |
| 800 | 32,8 | 193 | 453 | 1752 | 3162 |
| 900 | 32,8 | 192 | 453 | 1750 | 3161 |
| 1000 | 32,8 | 192 | 453 | 1746 | 3161 |
| 1500 | 32,7 | 192 | 453 | 1746 | 3143 |
| 2000 | 32,6 | 192 | 453 | 1746 | 3137 |
| 2200 ( = arrêt l'agitation) | 32,6 | 192 | 453 | 1746 | 3137 |
| 2800 ( = reprise l'agitation ) | 33,5 | 200 | 481 | 1974 | 3653 |
| 2900 | 32,6 | 198 | 465 | 1800 | 3197 |
| 3000 | 32,6 | 196 | 463 | 1782 | 3155 |

(suite)

|  | Exemple 1 | Exemple 2 | Exemple 3 | Exemple 4 | Exemple 5 |
|---|---|---|---|---|---|
| Temps (en sec) | Viscosité (en cP) | Viscosité (en cP) | Viscosité (en cP) | Viscosité (en cP) | Viscosité (en cP) |
| 3500 | 32,5 | 196 | 463 | 1770 | 3125 |
| 4000 | 32,5 | 196 | 463 | 1770 | 3113 |
|  |  |  |  |  |  |
| $\eta 0$ | 35,6 | 220 | 549 | 2525 | 4397 |
| $\eta 1$ | 32,6 | 192 | 453 | 1746 | 3137 |
| $\eta 2$ | 33,5 | 200 | 481 | 1974 | 3653 |

TABLEAU 2 :

|  | Exemple 1 | | Exemple 2 | | Exemple 3 | | Exemple 4 | | Exemple 5 | |
|---|---|---|---|---|---|---|---|---|---|---|
|  | % composition | | % composition | | % composition | | % composition | | % composition | |
| N° tampon | 1ere série | 2eme série | 1ere série | 2eme série | 1ere série | 2eme série | 1ere série | 2eme série | 1ere série | 2eme série |
| 1 | 34 | 29 | 40 | 33 | 41 | 33 | 76 | 38 | 97 | 48 |
| 2 | 29 | 27 | 30 | 26 | 33 | 29 | 24 | 33 | 3 | 36 |
| 3 | 20 | 21 | 21 | 21 | 21 | 22 | 0 | 21 | 0 | 15 |
| 4 | 12 | 15 | 8 | 13 | 5 | 12 | 0 | 7 | 0 | 1 |
| 5 | 5 | 8 | 1 | 7 | 0 | 4 | 0 | 1 | 0 | 0 |

TABLEAU 3:

|  | Exemple 1 | Exemple 2 | Exemple 3 | Exemple 4 | Exemple 5 |
|---|---|---|---|---|---|
| M1 (en g) | 2,17 | 2,08 | 2,13 | 2,13 | 2,19 |
| M3 (en g) | 0,44 | 0,42 | 0,43 | 0,43 | 0,44 |
| TI (en g/g) | 4,0 | 4,0 | 3,9 | 4,0 | 3,9 |
| M2 (10 sec) (en g) | 1,77 | 1,76 | 1,87 | 1,93 | 2,01 |
| M2 (30 sec) (en g) | 1,63 | 1,64 | 1,75 | 1,83 | 1,93 |
| M2 (60 sec) (en g) | 1,54 | 1,55 | 1,67 | 1,76 | 1,87 |
| M2 (120 sec) (en g) | 1,47 | 1,53 | 1,61 | 1,69 | 1,81 |
| TL (10 sec) (en %) | 22,9 | 19,1 | 15,5 | 11,8 | 10,0 |
| TL (30 sec) (en %) | 31,2 | 26,8 | 22,3 | 17,6 | 14,6 |
| TL (60 sec) (en %) | 36,3 | 31,7 | 26,9 | 21,5 | 18,5 |
| TL (120 sec) (en %) | 40,6 | 36,0 | 30,7 | 25,7 | 21,6 |

**Revendications**

1. Utilisation d'une composition de nettoyage et/ou de soin, comprenant au moins un composé de nettoyage et/ou de soin, ladite composition ayant une viscosité apparente mesurée à 20°C selon la norme ASTM D 2983 supérieure à 80 centipoises, de préférence supérieure à 200 centipoises, plus préférablement supérieure à 500 centipoises, encore plus préférablement supérieure à 2 500 centipoises, le plus préférablement supérieure à 12 500 centipoises, et étant rhéofluidifiante et thixotrope, pour l'imprégnation sous contrainte d'une pile de supports absorbants.

**2.** Utilisation selon la revendication 1 **caractérisée en ce que** ladite composition est assimilable à un corps plastique de Bingham jusqu'à ce que sa valeur atteigne une valeur seuil, lorsqu'elle est soumise à un taux de cisaillement donné.

**3.** Utilisation selon l'une quelconque des revendications précédentes **caractérisée en ce que** ladite composition a une viscosité η décroissant exponentiellement selon l'expression :

$$B = - (d\,\eta\,/\,d\,t).t,$$

le coefficient B étant supérieur ou égal à 0,4, de préférence supérieur ou égal à 4, plus préférablement compris entre 15 et 300 et encore plus préférablement compris entre 200 et 300,
lorsqu'elle est soumise à un taux de cisaillement de 20 tours/min au moyen d'un appareil Brookfield muni d'un mobile référence SC4-31 et jusqu'à l'obtention d'une valeur seuil de viscosité.

**4.** Utilisation selon l'une quelconque des revendications précédentes **caractérisée en ce que** ladite composition a un taux de reprise TR inférieur ou égal à 50 %, de préférence compris entre 20 % et 30 %.

**5.** Utilisation selon l'une quelconque des revendications précédentes **caractérisée en ce que** ladite composition comprend du magnésium aluminium silicate et du sodium carboxyméthylcellulose.

**6.** Utilisation selon l'une quelconque des revendications précédentes **caractérisée en ce que** ladite composition comprend au moins 0,4 %, de préférence au moins 0,7 %, plus préférablement au moins 0,8%, en poids, par rapport au poids total de ladite composition, de magnésium aluminium silicate et au moins 0,12 %, de préférence au moins 0,21 %, plus préférablement au moins 0,24 %, en poids, par rapport au poids total de ladite composition, de sodium carboxyméthylcellulose.

**7.** Utilisation selon l'une quelconque des revendications précédentes **caractérisée en ce que** le au moins un composé de nettoyage et/ou de soin est au moins un composé de nettoyage et/ou de soin de la peau.

**8.** Procédé de fabrication d'articles de nettoyage et/ou de soin **caractérisé en ce qu'**il comprend les étapes suivantes :

a) empilement l'un sur l'autre de plusieurs supports absorbants, et
b) application sous contrainte d'une composition de nettoyage et/ou de soin, comprenant au moins un composé de nettoyage et/ou de soin, ladite composition ayant une viscosité apparente mesurée à 20°C selon la norme ASTM D 2983 supérieure à 80 centipoises, de préférence supérieure à 200 centipoises, préférablement supérieure à 500 centipoises, plus préférablement supérieure à 2 500 centipoises, encore plus préférablement supérieure à 12 500 centipoises, et étant rhéofluidifiante et thixotrope sur le support absorbant disposé sur le haut de l'empilement.

**9.** Procédé selon la revendication 8 **caractérisé en ce qu'**à l'étape b), la composition de nettoyage et/ou de soin est assimilable à un corps plastique de Bingham jusqu'à ce que sa viscosité atteigne une valeur seuil.

**10.** Procédé selon l'une quelconque des revendications 8 ou 9 **caractérisé en ce qu'**à l'étape b), la composition de nettoyage et/ou de soin, lorsqu'elle est soumise à un taux de cisaillement de 20 tours/min au moyen d'un appareil Brookfield muni d'un mobile référence SC4-31 et jusqu'à l'obtention d'une valeur seuil de viscosité, a une viscosité η décroissant exponentiellement selon l'expression:

$$B = - (d\,\eta\,/\,d\,t).t,$$

le coefficient B étant supérieur ou égal à 0,4, de préférence supérieur ou égal à 4, plus préférablement compris entre 15 et 300, et encore plus préférablement compris entre 200 et 300.

**11.** Procédé selon l'une quelconque des revendications 8 à 10 caractérisé à ce qu'à l'étape b), la composition de soin et/ou de nettoyage possède un taux de reprise TR inférieur ou égal à 50 %, de préférence compris entre 20 et 30 %.

**12.** Procédé selon l'une quelconque des revendications 8 à 11 **caractérisé en ce qu'**à l'étape b), la composition de nettoyage et/ou de soin comprend du magnésium aluminium silicate et du sodium carboxyméthylcellulose.

**13.** Procédé selon l'une quelconque des revendications 8 à 12 **caractérisé en ce qu'**à l'étape b), la composition de soin et/ou de nettoyage comprend au moins 0,4 % en poids, de préférence au moins 0,7 % en poids, plus préférablement au moins 0,8 % en poids, par rapport au poids total de la composition, de magnésium aluminium silicate et au moins 0,12 % en poids, de préférence au moins 0,21 % en poids, plus préférablement au moins 0,24 % en poids, par rapport au poids total de la composition, de sodium carboxyméthylcellulose.

**Claims**

**1.** Use of a cleaning and/or care composition, comprising at least one cleaning and/or care compound, said composition having an apparent viscosity measured at 20°C in accordance with the standard ASTM D 2983 greater than 80 centipoises, preferably greater than 200 centipoises, more preferably greater than 500 centipoises, even more preferably greater than 2,500 centipoises, most preferably greater than 12,500 centipoises, and being rheofluidifying and thixotropic, for impregnating a stack of absorbent supports under constraint.

**2.** Use as claimed in claim 1, **characterised in that** said composition is comparable to a Bingham plastic until its value reaches a threshold value when it is subjected to a given shear rate.

**3.** Use as claimed in any one of the preceding claims, **characterised in that** said composition has an exponentially decreasing viscosity $\eta$ conforming to the equation:

$$B = (d\,\eta\,/\,d\,t).t,$$

the coefficient B being greater than or equal to 0.4, preferably greater than or equal to 4, more preferably ranging between 15 and 300 and even more preferably between 200 and 300,
when it is subjected to a shear rate of 20 revolutions/min by means of a Brookfield tester equipped with an SC4-31 reference spindle and until a viscosity threshold value is obtained.

**4.** Use as claimed in any one of the preceding claims, **characterised in that** said composition has a recovery rate TR of less than or equal to 50%, preferably ranging between 20 % and 30 %.

**5.** Use as claimed in any one of the preceding claims, **characterised in that** said composition comprises magnesium aluminium silicate and sodium carboxymethyl cellulose.

**6.** Use as claimed in any one of the preceding claims, **characterised in that** said composition comprises at least 0.4 %, preferably at
least 0.7 %, more preferably at least 0.8 %, by weight, relative to the total weight of said composition, of magnesium aluminium silicate and at least 0.12 %, preferably at least 0.21 %, more preferably at least 0.24 % by weight, relative to the total weight of said composition, of sodium carboxymethyl cellulose.

**7.** Use as claimed in any one of the preceding claims, **characterised in that** the at least one cleaning and/or care composition is at least one skin cleaning and/or care compound.

**8.** Method of manufacturing cleaning and/or care articles, **characterised in that** it comprises the following steps:

a) stacking several absorbent supports one on top of the other, and
b) applying, under constraint, a cleaning and/or care composition comprising at least one cleaning and/or care compound, said composition having an apparent viscosity measured at 20°C in accordance with the standard ASTM D 2983 greater than 80 centipoises, preferably greater than 200 centipoises, preferably greater than 500 centipoises, more preferably greater than 2,500 centipoises, most preferably greater than 12,500 centipoises, and being rheofluidifying and thixotropic on the absorbent support disposed at the top of the stack.

9. Method as claimed in claim 8, **characterised in that** at step b), the cleaning and/or care composition is comparable to a Bingham plastic until its viscosity reaches a threshold value.

10. Method as claimed in any one of claims 8 or 9, **characterised in that** at step b), when it is subjected to a shear rate of 20 revolutions/min by means of a Brookfield tester equipped with an SC4-31 reference spindle and until it reaches a threshold viscosity, the cleaning and/or care composition has an exponentially decreasing viscosity η conforming to the equation:

$$B = (d\,\eta\,/\,d\,t).t,$$

the coefficient B being greater than or equal to 0.4, preferably greater than or equal to 4, more preferably ranging between 15 and 300 and even more preferably between 200 and 300.

11. Method as claimed in any one of claims 8 to 10, **characterised in that** at step b), the cleaning and/or care composition has a recovery rate TR of less than or equal to 50 %, preferably ranging between 20 and 30 %.

12. Method as claimed in any one of claims 8 to 11, **characterised in that** at step b) the cleaning and/or care composition comprises magnesium aluminium silicate and sodium carboxymethyl cellulose.

13. Method as claimed in any one of claims 8 to 12, **characterised in that** the cleaning and/or care composition comprises at least 0.4 % by weight, preferably at least 0.7 % by weight, more preferably at least 0.8 %, by weight, relative to the total weight of said composition, of magnesium aluminium silicate and at least 0.12 % by weight, preferably at least 0.21 % by weight, more preferably at least 0.24 %, by weight, relative to the total weight of said composition, of sodium carboxymethyl cellulose.

**Patentansprüche**

1. Verwendung einer Reinigungs- und/oder Pflegezusammensetzung, umfassend wenigstens eine Reinigungs- und/ oder Pflegeverbindung, wobei die Zusammensetzung eine bei 20° gemäß der Norm ASTM D 2983 gemessene scheinbare Viskosität aufweist, welche größer als 80 Centipoise, bevorzugt größer als 200 Centipoise, stärker bevorzugt größer als 500 Centipoise, noch stärker bevorzugt größer als 2500 Centipoise, am stärksten bevorzugt größer als 12500 Centipoise ist sowie strukturviskos und thixotrop ist, für die Zwangsimprägnierung eines Stapels von absorbierenden Trägern.

2. Verwendung nach Anspruch 1, **dadurch gekennzeichnet, dass** die Zusammensetzung mit einem Bingham-Kunststoffkörper vergleichbar ist bis ihr Wert einen Schwellenwert annimmt, wenn sie einer gegebenen Scherrate ausgesetzt wird.

3. Verwendung nach einem der vorhergehenden Ansprüche, **dadurch gekennzeichnet, dass** die Zusammensetzung eine Viskosität η aufweist, welche exponentiell abnimmt gemäß dem Ausdruck:

$$B = -\left(d\eta\,/\,dt\right)t\ ,$$

wobei der Koeffizient B größer oder gleich 0,4, bevorzugt größer oder gleich 4, stärker bevorzugt zwischen 15 und 300 enthalten und noch stärker bevorzugt zwischen 200 und 300 enthalten ist,
wenn sie mittels einer Brookfield-Vorrichtung ausgesetzt wird, welche mit einer Spindelreferenz SC4-31 versehen ist, und bis zum Erreichen eines Viskositätsschwellenwerts einer Scherrate von 20 Umdrehungen pro Minute ausgesetzt wird.

4. Verwendung nach einem der vorhergehenden Ansprüche, **dadurch gekennzeichnet, dass** die Zusammensetzung eine Erholungsrate TR aufweist, welche kleiner oder gleich 50%, bevorzugt zwischen 20% und 30% enthalten ist.

**5.** Verwendung nach einem der vorhergehenden Ansprüche, **dadurch gekennzeichnet, dass** die Zusammensetzung Magnesium-Aluminium-Silikat und Natriumcarboxymethylcellulose umfasst.

**6.** Verwendung nach einem der vorhergehenden Ansprüche, **dadurch gekennzeichnet, dass** die Zusammensetzung wenigstens 0,4%, bevorzugt wenigstens 0,7%, stärker bevorzugt wenigstens 0,8% an Gewicht im Verhältnis zu dem Gesamtgewicht der Zusammensetzung von Magnesium-Aluminium-Silikat und wenigstens 0,12%, bevorzugt wenigstens 0,21%, stärker bevorzugt wenigstens 0,24% an Gewicht im Verhältnis zu dem Gesamtgewicht der Zusammensetzung von Natriumcarboxylmethylcellulose umfasst.

**7.** Verwendung nach einem der vorhergehenden Ansprüche, **dadurch gekennzeichnet, dass** die wenigstens eine Reinigungs - und/oder Pflegeverbindung wenigstens eine Verbindung zur Reinigung und/oder Pflege der Haut ist.

**8.** Verfahren zur Herstellung von Reinigungs- und/oder Pflegeartikeln, **dadurch gekennzeichnet, dass** es die folgenden Schritte umfasst:

a) Aufeinander Stapeln von mehreren absorbierenden Trägern, und

b) Anwenden unter Zwang einer Reinigungs- und/oder Pflegezusammensetzung, welche wenigstens eine Reinigungs - und/oder Pflegeverbindung umfasst, wobei die Zusammensetzung eine bei 20°C gemäß der Norm ASTM D 2983 gemessene scheinbare Viskosität aufweist, welche größer als 80 Centipoise, vorzugsweise größer als 200 Centipoise, bevorzugt größer als 500 Centipoise, stärker bevorzugt größer als 2500 Centipoise, noch stärker bevorzugt größer als 12500 Centipoise ist sowie strukturviskos und thixotrop ist, auf dem absorbierenden Träger, welcher auf der Oberseite des Stapels angeordnet ist.

**9.** Verfahren nach Anspruch 8, **dadurch gekennzeichnet, dass** bei dem Schritt b) die Reinigungs- und/oder Pflegezusammensetzung mit einem Bingham-Kunststoffkörper vergleichbar ist, bis ihre Viskosität einen Schwellenwert erreicht.

**10.** Verfahren nach einem der Ansprüche 8 oder 9, **dadurch gekennzeichnet, dass** bei dem Schritt b) die Reinigungs - und/oder Pflegezusammensetzung, wenn sie mittels einer Brookfield-Vorrichtung, welche mit einer Spindelreferenz SC4-31 versehen ist, und bis zum Erreichen eines Viskositätsschwellenwerts einer Scherrate von 20 Umdrehungen pro Minute ausgesetzt wird, eine Viskosität $\eta$ aufweist, welche exponentiell abnimmt gemäß dem Ausdruck:

$$B = -(d\eta / dt)t \ ,$$

wobei der Koeffizient B größer oder gleich 0,4, bevorzugt größer oder gleich 4, stärker bevorzugt zwischen 15 und 300 enthalten und noch stärker bevorzugt zwischen 200 und 300 enthalten ist.

**11.** Verfahren nach einem der Ansprüche 8 bis 10, **dadurch gekennzeichnet, dass** bei dem Schritt b) die Reinigungs - und/oder Pflegezusammensetzung eine Erholungsrate TR aufweist, welche kleiner oder gleich 50%, bevorzugt zwischen 20 und 30% enthalten ist.

**12.** Verfahren nach einem der Ansprüche 8 bis 11, **dadurch gekennzeichnet, dass** bei dem Schritt b) die Reinigungs - und/oder Pflegezusammensetzung Magnesium-Aluminium-Silikat und Natriumcarboxymethylcellulose umfasst.

**13.** Verfahren nach einem der Ansprüche 8 bis 12, **dadurch gekennzeichnet, dass** bei dem Schritt b) die Reinigungs - und/oder Pflegezusammensetzung wenigstens 0,4% an Gewicht, bevorzugt wenigstens 0,7% an Gewicht, stärker bevorzugt wenigstens 0,8% an Gewicht im Verhältnis zu dem Gesamtgewicht der Zusammensetzung von Magnesium-Aluminium-Silikat und wenigstens 0,12% an Gewicht, bevorzugt wenigstens 0,21% an Gewicht, stärker bevorzugt wenigstens 0,24% an Gewicht im Verhältnis zu dem Gesamtgewicht der Zusammensetzung von Natriumcarboxymethylcellulose umfasst.

## FIG.1

## FIG.2

## FIG.3

## FIG.4

FIG.5

**RÉFÉRENCES CITÉES DANS LA DESCRIPTION**

**Documents brevets cités dans la description**

- EP 0681621 A